(19) Europäisches Patentamt

European Patent Office

Office européen des brevets

(11) **EP 3 225 154 A2**

(12) **EUROPEAN PATENT APPLICATION**

(43) Date of publication:
04.10.2017 Bulletin 2017/40

(51) Int Cl.:
*A61B 3/113* (2006.01)

(21) Application number: 17158347.9

(22) Date of filing: 28.02.2017

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: 31.03.2016 JP 2016073481

(71) Applicant: **Fujitsu Limited
Kanagawa 211-8588 (JP)**

(72) Inventors:
• **Ishii, Daisuke
Kanagawa, 211-8588 (JP)**
• **Nakashima, Satoshi
Kanagawa, 211-8588 (JP)**

(74) Representative: **Hoffmann Eitle
Patent- und Rechtsanwälte PartmbB
Arabellastraße 30
81925 München (DE)**

(54) **GAZE DETECTION APPARATUS AND GAZE DETECTION METHOD**

(57)     A gaze detection apparatus includes: an image capturing unit which generates an image representing both eyes of a user; an eye detection unit which, for each of the eyes, detects an eye region containing the eye from the image; a pupil detection unit which, for one of the eyes, detects a pupil region containing a pupil from the eye region; an estimating unit which obtains, based on the pupil region detected for the one eye, an estimated shape of the pupil region on the image for the other one of the eyes; a redetection unit which detects the pupil region by searching for a region having a shape that matches the estimated shape from within the eye region detected for the other eye; and a gaze detection unit which detects the user's gaze direction based on a position of the pupil region detected for each of the eyes.

FIG. 1

EP 3 225 154 A2

## Description

FIELD

The embodiments discussed herein are related to a gaze detection apparatus and gaze detection method for detecting gaze direction based on an image captured of a human eye.

BACKGROUND

Work is underway to detect a person's eye gaze direction and thereby obtain information on an object attracting the person's attention or automatically execute an operation related to the gaze direction or the position at which the person is gazing. For convenience, the position being gazed at by a person will hereinafter be referred to simply as the gaze position.

A person's gaze position or gaze direction is closely associated with the direction in which a person's pupil is pointing. In view of this, it is known to provide techniques that detect a pupil from an image captured of a person's eye and identify the person's gaze direction or gaze position based on the positional relationship of the pupil relative to a reference point. For example, a corneal reflection image of a light source (hereinafter referred to as the Purkinje image) is used as the reference point.

However, in some cases, the Purkinje image may overlap the pupil, and the pupil may not be captured in its entirety in the eye image. When this happens, the pupil may not be detected accurately from the eye image. To address this, there has been proposed a technique that actively exploits information contained in the Purkinje image and thereby stably detects the pupil even when most of the pupil is hidden from view by the Purkinje image (for example, refer to Japanese Unexamined Patent Publication (Kokai) No. 2012-24154).

According to the pupil detection apparatus disclosed in Japanese Unexamined Patent Publication (Kokai) No. 2012-24154, a plurality of line segments each of a predetermined length are set with a reference point within the Purkinje image taken as one end, and a luminance evaluation score is calculated for each line segment based on a reference luminance and the luminance of each pixel on the line segment. Then, based on the calculated evaluation scores, the pupil detection apparatus identifies from the plurality of line segments the pupil's centerline passing through the center of the pupil image, and detects the pupil image based on the luminance condition in the vicinity of the pupil's centerline.

SUMMARY

However, the Purkinje image is not the only image that has the possibility of overlapping the pupil image in the eye image. For example, when the viewer is wearing eyeglasses, an image of a light source reflected by the eyeglasses may overlap the pupil image. There is also the possibility that images of a plurality of light sources may be reflected into the eye image. In such cases, the technique disclosed in Japanese Unexamined Patent Publication (Kokai) No. 2012-24154 may not be able to accurately detect the pupil.

In one aspect, an object of the present invention is to provide a gaze detection apparatus that can estimate the position of the pupil of a user's eye even when the pupil of the eye is partially hidden by another image in the captured image.

According to one embodiment, a gaze detection apparatus is provided. The gaze detection apparatus includes an image capturing unit which generates an image representing both eyes of a user; an eye detection unit which, for each of the eyes, detects an eye region containing the eye from the image; a pupil detection unit which, for one of the eyes, detects a pupil region containing a pupil from the eye region; an estimating unit which, based on the pupil region detected for the one eye, obtains an estimated shape of the pupil region on the image for the other one of the eyes; a pupil redetection unit which detects the pupil region by searching for a region having a shape that matches the estimated shape from within the eye region detected for the other eye; and a gaze detection unit which detects the user's gaze direction based on a position of the pupil region detected for each of the eyes.

BRIEF DESCRIPTION OF DRAWINGS

Figure 1 is a diagram illustrating the hardware configuration of a digital signage system implementing one embodiment of a gaze detection apparatus.

Figure 2 is a diagram illustrating one example of an arrangement of an illuminating light source, an infrared camera, and a display unit.

Figure 3 is a functional block diagram of a control unit for implementing a gaze detection process.

Figure 4 is a diagram illustrating one example of an estimated range.

Figure 5 is a diagram illustrating one example of the relationship between the illuminating light source and a user's gaze direction.

Figure 6 is a diagram illustrating one example of how the position of the estimated range is corrected.

Figure 7 is a diagram illustrating another example of a method for determining the position of the estimated range.

Figure 8 is a diagram illustrating one example of the relationship between the gaze direction and the shape of a pupil region.

Figure 9 is a diagram illustrating one example of the relationship between the gaze direction and the shape of the pupil region when the user's face is oriented at an angle relative to the line of sight of the infrared camera.

Figure 10 is an explanatory diagram providing an overview of how the position of the pupil's centroid is estimated based on the contour of an unhidden portion of the pupil region.

Figure 11 is a diagram illustrating one example of a mapping table.

Figure 12 is a diagram illustrating one example of a gaze position table.

Figure 13 is a diagram illustrating an operation flowchart of the gaze detection process.

DESCRIPTION OF EMBODIMENTS

A gaze detection apparatus will be described below with reference to the drawings. The gaze detection apparatus generates an image by capturing, using a camera, both eyes of a user illuminated with a light source, and detects the gaze direction of the user by detecting the pupils of the respective eyes on the image. Then, based on the positional relationship of the detected pupil of one eye relative to the reference point preset for each eye, the gaze detection apparatus sets an estimated range within which the pupil is estimated to be located in the other eye. Further, based on the shape of the pupil detected for the one eye on the image, the gaze detection apparatus obtains an estimated shape of the pupil for the other eye on the image. Then, based on the estimated range and the estimated shape of the pupil, the gaze detection apparatus once again performs processing for detecting the pupil of the other eye.

In the embodiment hereinafter described, the gaze detection apparatus is mounted in a digital signage system, and detects the gaze position of the user of the digital signage system.

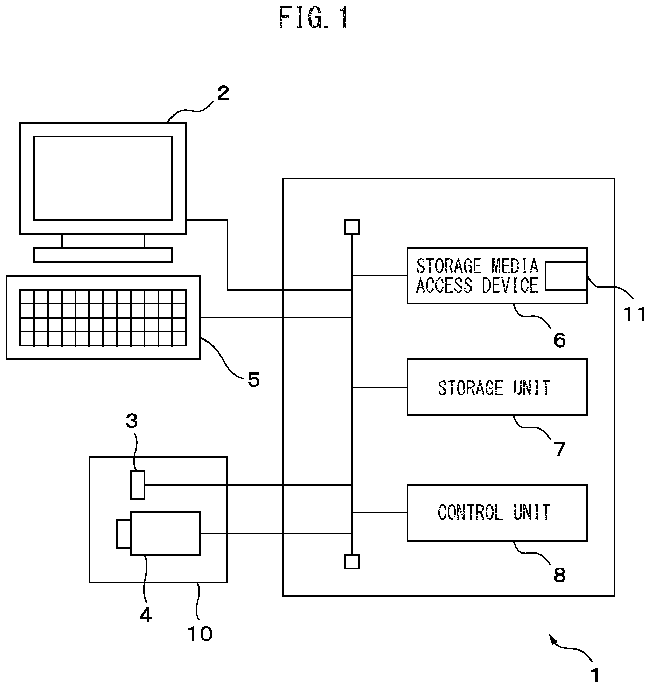

Figure 1 is a diagram illustrating the hardware configuration of the digital signage system implementing one embodiment of the gaze detection apparatus. The digital signage system 1 includes a display unit 2, an illuminating light source 3, an infrared camera 4, an input unit 5, a storage media access device 6, a storage unit 7, and a control unit 8. The digital signage system 1 may further include a communication interface circuit (not depicted) for connecting the digital signage system 1 to another apparatus.

The display unit 2 includes, for example, a liquid crystal display or an organic electroluminescent display. The display unit 2 displays, for example, various kinds of text, icons, still images, or moving images in accordance with control signals from the control unit 8.

The illuminating light source 3 includes an infrared light emitting source constructed, for example, from at least one infrared emitting diode, and a driving circuit (not depicted) which supplies power from a power supply (not depicted) to the infrared emitting diode in accordance with a control signal from the control unit 8. The illuminating light source 3 is mounted in the same cabinet 10 as the infrared camera 4 with the light emitting face of the illuminating light source 3 oriented in the same direction as the shooting direction of the infrared camera 4 so as to be able to illuminate the shooting direction of the infrared camera 4. The illuminating light source 3 continues to emit the illuminating light during the period when the control signal for lighting the light source is being received from the control unit 8, for example.

The infrared camera 4 is one example of an image capturing unit, and generates an image containing at least a portion of the user's face including the eyes. For this purpose, the infrared camera 4 includes an image sensor constructed from a two-dimensional array of solid-state imaging devices having sensitivity to the infrared light radiated from the illuminating light source 3, and an imaging optical system for focusing an image of the subject onto the image sensor. The infrared camera 4 may further include a visible-light cutoff filter between the image sensor and the imaging optical system in order to prevent an image reflected by the iris and a Purkinje image of any light other than the light from the illuminating light source 3 from being detected. The imaging optical system may be a fixed focus optical system, or may alternatively be a variable focus optical system. During the execution of the gaze detection process, the infrared camera 4 generates images by shooting images at a certain frame rate. The infrared camera 4 has a resolution high enough that the pupil and the Purkinje image of the illuminating light source 3 reflected on the cornea of the user of the digital signage system 1 can be recognized in the generated image. Each time such an image is generated, the infrared camera 4 passes the image to the control unit 8.

In the present embodiment, the illuminating light source 3 and the infrared camera 4 are mounted in the same cabinet 10. The cabinet 10 may include a rotatable supporting member (not depicted) rotatably mounted on the surface of the mounting base of the cabinet 10 while supporting the illuminating light source 3 and the infrared camera 4 in an integral fashion, and an actuator (not depicted) that rotates the rotatable supporting member in accordance with a control signal from the control unit 8. In this way, the infrared camera 4 is mounted in the cabinet 10 so as to be able to change its shooting direction.

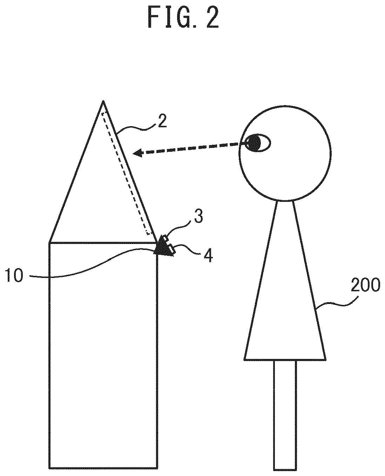

Figure 2 is a diagram illustrating one example of an arrangement of the illuminating light source 3, the infrared camera 4, and the display unit 2. The cabinet 10 in which the illuminating light source 3 and the infrared camera 4 are mounted is located near the display unit 2. The illuminating light source 3 and the infrared camera 4 are oriented toward the user 200. The infrared camera 4 captures an image of a portion or the entire area of the face of the user 200 including the two eyes of the user 200.

The input unit 5 includes, for example, a keyboard and a pointing device such as a mouse. An operation signal entered via the input unit 5 by the user is passed to the control unit 8.

The display unit 2 and the input unit 5 may be combined into one unit, for example, as in the case of a touch panel display. In this case, when the user touches an icon displayed at a specific position on the display screen of the display unit 2, the input unit 5 generates an operation signal associated with that position and supplies the operation signal to the control unit 8.

The storage media access device 6 is a device that accesses a storage medium 11 such as a magnetic disk, a semiconductor memory card, or an optical storage medium. The storage media access device 6 accesses the storage medium 11 to read, for example, a gaze detection computer program to be executed on the control unit 8, and passes the program to the control unit 8.

The storage unit 7 includes, for example, a readable/writable nonvolatile semiconductor memory and a readable/writable volatile semiconductor memory. The storage unit 7 stores the gaze detection computer program and various application programs to be executed on the control unit 8 and various kinds of data for the execution of the programs.

The storage unit 7 further stores various kinds of data to be used for the detection of the user's gaze position. For example, the storage unit 7 stores a mapping table that provides a mapping between the gaze direction and the position of the centroid of the pupil relative to the centroid of the Purkinje image.

The control unit 8 includes one or a plurality of processors and their peripheral circuitry. The control unit 8 is connected to each unit of the digital signage system 1 by a signal line, and controls the entire operation of the digital signage system 1. For example, the control unit 8 performs processing appropriate to the operation signal received from the input unit 5 and the application program currently being executed, and displays moving images, etc., on the display unit 2.

Further, each time the image and a narrow-angle image are acquired, the control unit 8 detects the user's gaze direction and gaze position by executing the gaze detection process.

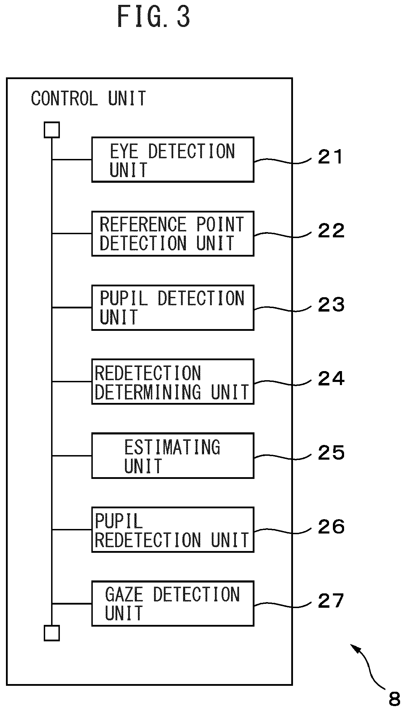

Figure 3 is a functional block diagram of the control unit 8 for implementing the gaze detection process. The control unit 8 includes an eye detection unit 21, a reference point detection unit 22, a pupil detection unit 23, a redetection determining unit 24, an estimating unit 25, a pupil redetection unit 26, and a gaze detection unit 27. These units constituting the control unit 8 are functional modules implemented by executing a computer program on a processor incorporated in the control unit 8. Alternatively, these units constituting the control unit 8 may be implemented on a single or a plurality of integrated circuits on which the circuits corresponding to the respective units are integrated, and may be mounted in the digital signage system 1 separately from the processor incorporated in the control unit 8.

Each time an image is acquired during the execution of the gaze detection process, the eye detection unit 21 detects an eye region containing the user's eye on the image for each of the user's left and right eyes. Since the eye detection unit 21 repeats the same processing for all images, the processing performed for one image will be described below.

For example, when it is assumed that the entire area of the user's face is contained in the image, first the eye detection unit 21 detects a face region containing the user's face from the image. For this purpose, the eye detection unit 21 converts the value of each pixel in the image, for example, into a value defined by the HSV color system. Then, the eye detection unit 21 extracts any pixel whose hue component (H component) value falls within the range of values corresponding to skin tones (for example, the range of values from 0° to 30°) as a face region candidate pixel that potentially corresponds to a portion of the face.

Further, when the user is using the digital signage system 1, it can be assumed that the user's face is oriented toward the display unit 2 and is located at a certain distance (for example, one to two meters) from the display unit 2. As a result, the size of the region that the face occupies on the image can be estimated to a certain extent.

Therefore, the eye detection unit 21 performs labeling on the face region candidate pixels, and extracts a set of neighboring face region candidate pixels as a face candidate region. Then, the eye detection unit 21 determines whether the size of the face candidate region falls within a reference range corresponding to the size of the user's face. When the size of the face candidate region falls within the reference range corresponding to the size of the user's face, the eye detection unit 21 determines that the face candidate region is the face region.

The eye detection unit 21 may use not only the size of the face candidate region but also the shape of the face candidate region as the criteria for determining whether the face candidate region is the face region or not. Generally, a human face is substantially elliptical in shape. In view of this, when the size of the face candidate region falls within the above reference range, and when the ellipticity of the face candidate region is not less than a given threshold value corresponding to the contour of a typical face, for example, the eye detection unit 21 may determine that the face candidate region is the face region. In this case, the eye detection unit 21 can calculate the ellipticity by obtaining the total number of pixels located on the contour of the face candidate region as the circumferential length of the face candidate region, multiplying the total number of pixels contained in the face candidate region by $4\pi$, and dividing the result by the square of the circumferential length.

Alternatively, the eye detection unit 21 may approximate the face candidate region by an ellipse by substituting the coordinates of each pixel on the contour of the face candidate region into an elliptic equation and by applying a least square method. Then, when the ratio of the major axis to the minor axis of the ellipse falls within a range defining the minimum and maximum of the ratio of the major axis to the minor axis of a typical face, the eye detection unit 21 may determine that the face candidate region is the face region. When evaluating the shape of the face candidate region by an elliptic approximation, the eye detection unit 21 may detect edge pixels corresponding to edges by calculating differences in luminance between adjacent pixels in the image. In this case, the eye detection unit 21 connects the edge pixels, for example, by using a technique of labeling, and determines that the edge pixels with a connected length longer than a predetermined length represents the contour of the face candidate region.

Alternatively, the eye detection unit 21 may detect the face region by using any one of various other methods for detecting the region of the face contained in the image. For example, the eye detection unit 21 may perform template matching between the face candidate region and a template corresponding to the shape of a typical face and calculate the degree of matching between the face candidate region and the template; then, when the degree of matching is higher than a predetermined value, the eye detection unit 21 may determine that the face candidate region is the face region.

Further alternatively, the eye detection unit 21 may detect the face region from the image by using a classifier pretrained to detect a face region from an image. In this case, AdaBoost or Real AdaBoost, Support Vector Machine, or Deep Neural Network, for example, is used as the classifier. Then, the eye detection unit 21 sets a window on the image, extracts the values of the pixels contained in the window or extracts features from the window while changing the position of the window, and determines whether the window contains the face region by entering the extracted values or features into the classifier. Haar-like features or Histograms of Oriented Gradients features, for example, are extracted as the features.

The eye detection unit 21 detects an eye region for each of the left and right eyes from the face region.

The luminance of the pixels corresponding to the eye greatly differs from the luminance of the pixels corresponding to the peripheral region of the eye. In view of this, the eye detection unit 21 calculates differences between vertically neighboring pixels in the face region by applying, for example, Sobel filtering, and detects edge pixels between which the luminance changes in the vertical direction. Then, the eye detection unit 21 detects, for example, a region bounded by two edge lines each formed by connecting a predetermined number of edge pixels corresponding to the size of the eye in a substantially horizontal direction, and takes such a region as an eye region candidate.

The two eyes of a human are arranged one spaced apart from the other in the horizontal direction. In view of this, the eye detection unit 21 extracts, from among the detected eye region candidates, two eye region candidates for which the difference in position between the centroids as measured in the vertical direction is the smallest, and which are separated from each other in the horizontal direction by a distance corresponding to the distance between the left and right eyes. Then, the eye detection unit 21 determines that the two eye region candidates each represent the eye region.

Alternatively, the eye detection unit 21 may perform template matching between the face region and a template representing the eye to detect from within each of the left and right halves of the face region a region that best matches the template, and may then determine that the detected region is the eye region. Further alternatively, the eye detection unit 21 may detect the eye region from the face region by using a classifier pretrained to detect an eye region from an image. In this case also, AdaBoost or Real AdaBoost, Support Vector Machine, or Deep Neural Network, for example, is used as the classifier. Then, the eye detection unit 21 sets an attention window within the face region, extracts the values of the pixels contained in the window or extracts features from the window, and determines whether the window contains the eye region by entering the extracted values or features into the classifier.

Further alternatively, the eye detection unit 21 may detect the face region and the left and right eye regions simultaneously by applying a deformable part model method to the image. For the deformable part method, refer, for example, to "A Discriminatively Trained, Multiscale, Deformable Part Model," by P. Felzenszwalb, et al., Computer Vision and Pattern Recognition 2008, IEEE Conference on Year, pp. 1-8, 2008.

When only a portion of the face is contained in the image, the eye detection unit 21 may skip the face region detection process. Then, the eye detection unit 21 may detect the eye region for each of the left and right eyes by performing any one of the above eye region detection processes over the entire image.

The eye detection unit 21 passes information representing the eye region for each of the left and right eyes to the reference point detection unit 22 and the pupil detection unit 23.

Each time the information representing the eye region is received from the eye detection unit 21, the reference point detection unit 22 detects, from the image for which the information representing the eye region has been obtained, a reference point which is referred to when redetecting the pupil for each of the left and right eyes and whose position does not change with the gaze direction. Since the reference point detection unit 22 repeats the same processing for each of the left and right eyes, the processing performed for one eye will be described below.

In the present embodiment, the reference point detection unit 22 sets the reference point based on the Purkinje image of the illuminating light source 3. The luminance of the region containing the Purkinje image of the illuminating light source 3 is higher than the luminance of its surrounding region, and its luminance value is substantially saturated (i.e., the luminance value is substantially equal to the highest luminance value that the pixel value can take). Further, the shape of the region containing the Purkinje image of the illuminating light source 3 substantially coincides with the shape of the light-emitting face of the illuminating light source 3. In view of this, the reference point detection unit 22 sets, within the eye region, two rings having a common center but differing in size and having a shape that substantially coincides with the contour shape of the light-emitting face of the illuminating light source 3. Then, the reference point detection unit 22 obtains a difference value by subtracting the average luminance value of the outer pixels from the inner luminance average value representing the average luminance value of the pixels corresponding to the inner ring. Then, when the difference value is larger than a predetermined difference threshold value, and when the inner luminance average value is higher than a predetermined luminance threshold value, the reference point detection unit 22 determines that the region enclosed by the inner ring represents the Purkinje image of the illuminating light source 3. The difference threshold value may be set, for example, equal to the average value of the difference values calculated between neighboring pixels in the eye region. On the other hand, the predetermined luminance threshold value may be set, for example, to 80% of the highest luminance value in the eye region.

The reference point detection unit 22 may detect the region containing the Purkinje image of the illuminating light source 3 by using any one of various other methods for detecting the region containing the Purkinje image of the light source on the image.

The reference point detection unit 22 takes as the reference point the centroid of the region containing the Purkinje image.

According to a modified example, the reference point detection unit 22 may detect the reference point from the shape of the eye itself. For example, the reference point detection unit 22 may detect the inner corner of the eye, the outer corner of the eye, or the midpoint between the inner and outer corners of the eye as the reference point. Alternatively, the reference point detection unit 22 may partition the eye region in a gridlike pattern with horizontal and vertical bars arranged at a predetermined pitch, and may set any one of the intersections of the horizontal and vertical bars as the reference point.

For example, when detecting the inner corner of the eye as the reference point, the reference point detection unit 22 applies corner detection filtering around the eye region to detect a convex corner to the center of the face as the inner corner of the eye. Alternatively, the reference point detection unit 22 may detect the inner corner of the eye by performing template matching using a template representing the inner corner of the eye around the eye region. Further alternatively, the reference point detection unit 22 may detect the inner corner of the eye by using a classifier pretrained to detect the inner corner of an eye. Likewise, when detecting the outer corner of the eye as the reference point, the reference point detection unit 22 applies corner detection filtering around the eye region to detect a convex corner to the outside of the face as the outer corner of the eye. Alternatively, the reference point detection unit 22 may detect the outer corner of the eye by performing template matching using a template representing the outer corner of the eye around the eye region. Further alternatively, the reference point detection unit 22 may detect the outer corner of the eye by using a classifier pretrained to detect the outer corner of an eye.

On the other hand, when detecting the midpoint between the inner and outer corners of the eye as the reference point, the reference point detection unit 22 detects the inner and outer corners of the eye as described above, and obtains the midpoint between them.

The reference point detection unit 22 calculates the coordinates representing the position of the reference point on the image for each of the left and right eyes, and passes the coordinates to the estimating unit 25, the pupil redetection unit 26, and the gaze detection unit 27.

Each time the information representing the eye region is received from the eye detection unit 21, the pupil detection unit 23 detects, from the image for which the information representing the eye region has been obtained, a pupil region containing the pupil for each of the left and right eyes. Since the pupil detection unit 23 repeats the same processing for each of the left and right eyes, the processing performed for one eye will be described below.

In the present embodiment, the pupil detection unit 23 performs template matching between the eye region and a template representing the pupil, and detects from within the eye region a region that best matches the template. Then, when the maximum value of the degree of matching is higher than a predetermined degree-of-matching threshold value, the pupil detection unit 23 determines that the detected region is the pupil region containing the pupil. The pupil size changes with the amount of light entering the user's eye. The pupil is substantially circular in shape, but when the infrared camera 4 is directed at an oblique angle to the pupil, the shape of the pupil on the image is a vertically elongated ellipse. In view of this, a plurality of templates may be prepared according to the size or shape of the pupil. In this case, the pupil detection unit 23 matches the eye region against the plurality of templates, and obtains the maximum value of the degree of matching. When the maximum value of the degree of matching is higher than the degree-of-matching threshold value, the pupil detection unit 23 determines that the pupil is contained in the region that matches the template corresponding to the maximum value of the degree of matching. The degree of matching is calculated, for example, as the value of normalized cross-correlation between the template and the region that matches the template. The degree-of-matching threshold value is set, for example, to 0.7 or 0.8.

The luminance of the region containing the pupil is lower than the luminance of its surrounding region, and the pupil is substantially circular in shape. In view of this, the pupil detection unit 23 sets a double ring filter having two concentric rings of different radii within the eye region. Then, when the difference value obtained by subtracting the average luminance value of the inner pixels from the average luminance value of the pixels corresponding to the outer ring is larger than a predetermined threshold value, the pupil detection unit 23 may determine that the region enclosed by the inner ring represents the pupil region. The pupil detection unit 23 may detect the pupil region by further detecting that the average luminance value of the region enclosed by the inner ring is not larger than a predetermined threshold value. In this case, the predetermined threshold value is set equal to a value obtained, for example, by adding 10 to 20% of the difference between the maximum and minimum luminance values of the eye region to the minimum luminance value.

Alternatively, the pupil detection unit 23 may detect the pupil region by using a classifier, such as AdaBoost or Support Vector Machine, that has been pretrained for pupil region detection. Further alternatively, the pupil detection unit 23 may detect the pupil region by utilizing the fact that the color or luminance distribution in the pupil region is different from the color or luminance distribution in its surrounding region. In this case, the pupil detection unit 23 may obtain, for example, using a region growing method, an aggregate of pixels having luminance values falling within a predetermined range of luminance values starting from the minimum luminance value, including the pixel having the minimum luminance value within the eye region, and may detect the aggregate of such pixels as the pupil region.

Further, the pupil detection unit 23 may detect the region containing the pupil by using any one of various other methods for detecting the region of the pupil contained in the image.

When the pupil region has been detected successfully, the pupil detection unit 23 calculates the position coordinates of the centroid of the pupil region (hereinafter referred to as the pupil's centroid) by taking the average value of the horizontal coordinate values and the average value of the vertical coordinate values of the pixels contained in the pupil region.

The pupil detection unit 23 supplies to the redetection determining unit 24 information indicating whether or not the pupil region has been detected successfully for each of the left and right eyes and the evaluation score, calculated at the time of the detection of the pupil region, that indicates the degree of certainty of the pupil region. For example, when template matching is used for the detection the pupil region, the evaluation score is the maximum value of the degree of matching. When a double ring filter is used for the detection of the pupil region, the difference value, i.e., the output value of the double ring filter at the position where the double ring filter is located when the pupil region is detected, can be taken as the evaluation score. On the other hand, when a classifier is used for the detection of the pupil region, the output value of the classifier, which is compared with a threshold value for identifying whether the detected region is the pupil region or not, may be used as the evaluation score. When the pupil region is detected using a region growing method, the ellipticity of the pupil region may be used as the evaluation score. For the eye for which the pupil region has been detected successfully, the pupil detection unit 23 supplies the position coordinates of the pupil's centroid to both the estimating unit 25 and the gaze detection unit 27 and the information representing the pupil region to the estimating unit 25.

Each time the information indicating whether or not the pupil region has been detected successfully and the evaluation score indicating the degree of certainty of the pupil region are received for each of the left and right eyes, the redetection determining unit 24 determines which of the eyes is to be redetected.

For example, when the pupil region has been detected successfully for one of the left and right eyes, but the detection of the pupil region failed for the other eye, the redetection determining unit 24 determines that the eye for which the detection of the pupil region failed is the eye for which the pupil region is to be redetected.

On the other hand, when the pupil region detection has been done successfully for both of the left and right eyes, the redetection determining unit 24 determines that the eye with the lower evaluation score is the eye for which the pupil region is to be redetected.

However, when the pupil region detection has been done successfully for both of the left and right eyes, when the evaluation scores for both of the left and right eyes are not lower than a predetermined threshold value, then the redetection determining unit 24 may determine that the redetection of the pupil region is not needed for either of the eyes. This is because it can be presumed that the pupil regions for both eyes have been detected with a relatively high accuracy. When the pupil region detection failed for both of the eyes, the redetection determining unit 24 may determine that the detection of the gaze direction and gaze position need not be performed on the image.

The redetection determining unit 24 supplies information identifying the eye for which the pupil region is to be redetected to the estimating unit 25 and the pupil redetection unit 26.

Each time the information identifying the eye for which the pupil region is to be redetected and the information representing the pupil region is received, the estimating unit 25, based on the pupil region and the reference point for the eye for which the pupil region need not be redetected, sets redetection conditions, i.e., the detection conditions for redetecting the pupil region for the other eye. In the following description, the eye for which the pupil region is to be redetected is referred to as the redetection target eye, and the eye for which the pupil region need not be redetected is referred to as the reference eye.

In the present embodiment, the estimating unit 25 sets, as the redetection conditions, an estimated range within which the pupil region is estimated to be located and an estimated shape of the pupil region to be detected.

For the purpose, the estimating unit 25 first obtains the horizontal width (i.e., the width measured in the direction along which the two eyes are arranged) and vertical width (i.e., the width measured in the direction perpendicular to the direction along which the two eyes are arranged) of the pupil region for the reference eye. For example, when the pupil is detected by template matching, the estimating unit 25 determines that the horizontal width and vertical width of the pupil in the template that best matches the pupil represent respectively the horizontal width and vertical width of the pupil. Further, in order that the shape of the pupil can be obtained with higher accuracy, the estimating unit sets, for example, a plurality of lines radially extending from the pupil's centroid, and detects on each line an edge whose luminance value is lower on the side nearer to the pupil's centroid. The estimating unit 25 may then obtain the contour shape of the pupil by connecting the detected edges, for example, in accordance with a contour extraction method using a dynamic contour model such as Snake. Then, based on the contour shape of the pupil, the estimating unit 25 may calculate the horizontal width and the vertical width.

To set the estimated range for the pupil region, the estimating unit 25 calculates, for example, a vector describing the direction and distance from the reference point to the pupil's centroid for the reference eye. When the reference point is given as the Purkinje image or the midpoint between the inner and outer corners of the eye, the estimating unit 25 sets the estimated range so that its center is located at the point indicated by the vector from the reference point for the redetection target eye. On the other hand, when the reference point is a specific point, such as the inner corner or outer corner of the eye, such that the position of the reference point relative to the pupil is reversed between the left and right eyes, the estimating unit 25 reverses horizontally the direction indicated by the vector calculated for the reference eye. After that, the estimating unit 25 sets the estimated range so that its center is located at the point indicated by the reversed vector from the reference point for the redetection target eye.

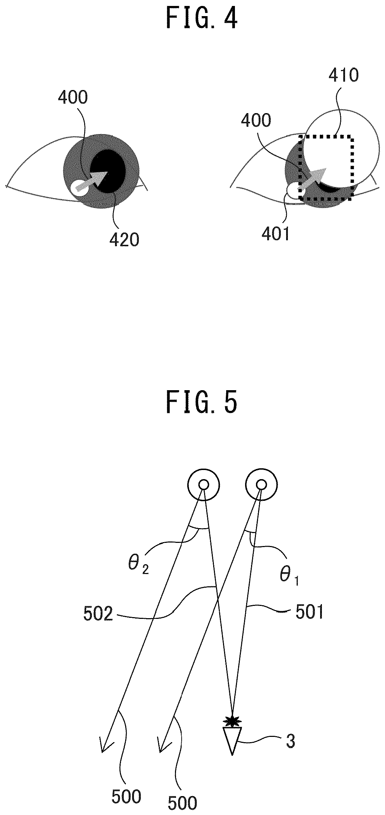

Figure 4 is a diagram illustrating one example of the estimated range. In the illustrated example, it is assumed that the pupil region is to be redetected for the eye located on the right side in the image (i.e., the user's left eye). In this case, the position 402 indicated by the vector 400 from the reference point for the right-side eye, the vector 400 being the same as the vector 400 from the reference point for the eye located on the left side in the image to the pupil's centroid, is the center of the estimated range 410. On the other hand, the difference between the positions of the user's left and right eyes in the vertical direction can be considered small. Therefore, the estimating unit 25 sets the vertical width of the estimated range 410 either equal to the vertical width of the pupil region 420 of the left-side eye for which the pupil region is not to be redetected or equal to the vertical width of the pupil region plus a relatively small offset (for example, two to five pixels). Further, the estimating unit 25 sets the horizontal width of the estimated range 410 equal to the width of the pupil region 420 plus a relatively large offset (for example, 5 to 20 pixels).

When the reference point is given as the Purkinje image of the illuminating light source 3, the estimating unit 25 may correct the position of the estimated range based on the positional relationship between the Purkinje image and the pupil's centroid for the reference eye.

Figure 5 is a diagram illustrating one example of the relationship between the illuminating light source 3 and the user's gaze direction. Generally, the position of the Purkinje image does not change with the user's gaze direction. On the other hand, the position of the pupil's centroid changes with the user's gaze direction. Further, the surface of the cornea is substantially spherical in shape. As a result, the relative positional relationship between the Purkinje image and the pupil's centroid changes with the user's gaze direction. Further, the angle that the gaze direction makes with the line joining the illuminating light source 3 to the Purkinje image differs between the right eye and the left eye.

For example, as illustrated in Figure 5, it is assumed that the illuminating light source 3 is located rightwardly of the user's gaze direction 500. In this case, the angle $\theta_2$ that the gaze direction 500 makes with the line 502 joining the illuminating light source 3 to the Purkinje image on the left-side eye is larger than the angle $\theta_1$ that the gaze direction 500 makes with the line 501 joining the illuminating light source 3 to the Purkinje image on the right-side eye. In other words, the distance between the pupil's centroid and the Purkinje image on the left-side eye is greater than the distance between the pupil's centroid and the Purkinje image on the right-side eye. Conversely, when the illuminating light source 3 is located leftwardly of the user's gaze direction, the angle that the gaze direction makes with the line joining the illuminating light source 3 to the Purkinje image on the right-side eye is larger than the angle that the gaze direction makes with the line joining the illuminating light source 3 to the Purkinje image on the left-side eye. In other words, the distance between the pupil's centroid and the Purkinje image on the right-side eye is greater than the distance between the pupil's centroid and the Purkinje image on the left-side eye.

In view of the above, the estimating unit 25 determines whether the illuminating light source 3 is located leftwardly or rightwardly of the user's gaze direction, for example, based on the positional relationship between the pupil's centroid and the Purkinje image. When the user is looking at the illuminating light source, the Purkinje image substantially coincides with the pupil's centroid. Accordingly, when the pupil's centroid is located leftwardly of the Purkinje image, the gaze direction is displaced rightwardly of the illuminating light source 3; on the other hand, when the pupil's centroid is located rightwardly of the Purkinje image, the gaze direction is displaced leftwardly of the illuminating light source 3. Then, when the positional relationship of the gaze direction relative to the illuminating light source 3 as viewed in the horizontal direction is identical to the positional relationship of the redetection target eye relative to the reference eye as viewed in the horizontal direction on the image, the estimating unit 25 shifts the position of the estimated range horizontally by a predetermined amount of correction in a direction directed away from the illuminating light source 3 and toward the gaze direction. Conversely, when the positional relationship of the gaze direction relative to the illuminating light source 3 as viewed in the horizontal direction is opposite to the positional relationship of the redetection target eye relative to the reference eye as viewed in the horizontal direction on the image, the estimating unit 25 shifts the position of the estimated range horizontally by a predetermined amount of correction in a direction directed away from the gaze direction and toward the illuminating light source 3.

For example, when the user's gaze direction 500 is displaced leftwardly of the illuminating light source 3, as depicted in Figure 5, it is assumed that the redetection target eye is the eye located on the left side in the image. In this case, the estimating unit 25 shifts the estimated range to the left relative to the position of the estimated range that is set based on the vector directed from the Purkinje image to the pupil's centroid on the right-side eye. Conversely, when the redetection target eye is the eye located on the right side in the image, the estimating unit 25 shifts the estimated range to the right relative to the position of the estimated range that is set based on the vector directed from the Purkinje image to the pupil's centroid on the left-side eye. On the other hand, when the user's gaze direction is displaced rightwardly of the illuminating light source 3, it is assumed that the redetection target eye is the eye located on the right side in the image. In this case, the estimating unit 25 shifts the estimated range to the right relative to the position of the estimated range that is set based on the vector directed from the Purkinje image to the pupil's centroid on the left-side eye. Conversely, when the redetection target eye is the eye located on the left side in the image, the estimating unit 25 shifts the estimated range to the left relative to the position of the estimated range that is set based on the vector directed from the Purkinje image to the pupil's centroid on the right-side eye.

The predetermined amount of correction is set in advance in accordance, for example, with the gaze direction. For example, the predetermined amount of correction is set larger as the angle that the gaze direction makes with the line joining the illuminating light source 3 to the Purkinje image is larger. Then, a position correction table providing a mapping between the gaze direction and the predetermined amount of correction, for example, is stored in advance in the storage unit 7. The estimating unit 25 can detect the gaze direction for the reference eye by performing processing similar to that performed by the gaze detection unit 27. Then, by referring to the position correction table, the estimating unit 25 determines the amount of correction that matches the gaze direction.

The estimating unit 25 may utilize the immediately previously obtained gaze direction as the gaze direction for the reference eye.

On the other hand, when the reference point is a specific point, such as the inner corner or outer corner of the eye, such that the position of the reference point relative to the pupil is reversed between the left and right eyes, the estimating unit 25 may correct the position of the estimated range based on the distance between the reference point and the pupil's centroid for the reference eye.

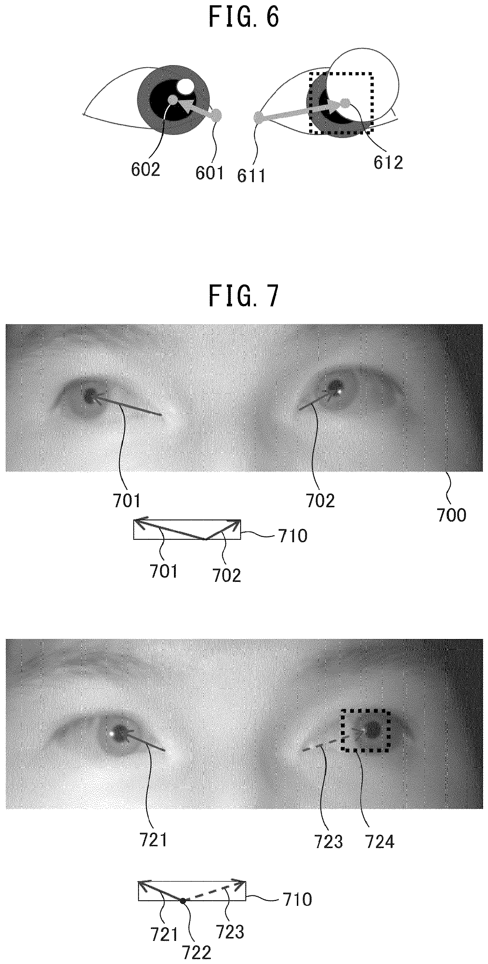

Figure 6 is a diagram illustrating one example of how the position of the estimated range is corrected in the above case. In the example illustrated in Figure 6, it is assumed that the eye located on the right side in the image is the redetection target eye. It is also assumed that the inner corner of the eye is used as the reference point. In this case, the estimating unit 25 increases the distance measured horizontally from the reference point (inner corner) 611 to the center of the estimated range 612 for the right-side eye as the distance from the reference point (inner corner) 601 to the pupil's centroid 602 for the left-side eye, i.e., the reference eye, decreases.

According to a modified example, as illustrated in Figure 7, vectors 701 and 702, each from the inner corner of the eye to the pupil's centroid, are detected for the respective eyes, based on a reference image 700 in which the pupil region is known. Then, a rectangular region 710, whose horizontal width is equal to the sum of the horizontal components of the vectors 701 and 702, and whose vertical width is equal to the average value of the vertical components of the vectors 701 and 702, may be stored in advance in the storage unit 7. A plurality of such rectangular regions 710 may be set according to the expected distance from the infrared camera 4 to the user and an installation condition such as the height of the infrared camera 4. Then, the estimating unit 25 may use the rectangular region that closely matches the expected distance and the installation condition.

In this case, the estimating unit 25 places the vector 701 within the rectangular region 710 so that the end point of a vector 721 directed from the reference point to the pupil's centroid on the reference eye coincides with one corner of the rectangular region 710. Then, the estimating unit 25 calculates a vector 723 directed from the starting point 722 of the vector 721 to another corner of the rectangular region 710 on the side horizontally opposite from the corner at which the end point of the vector 721 is located, and sets this vector as the vector from the inner corner of the eye, i.e., the reference point, to the center of the estimated range 724 for the other eye.

When the reference point is the midpoint between the inner and outer corners of the eye, when the positional relationship of the pupil's centroid relative to the reference point for the reference eye as viewed in the horizontal direction is identical to the positional relationship of the redetection target eye relative to the reference eye as viewed in the horizontal direction on the image, the estimating unit 25 may shift the position of the estimated range horizontally toward the reference point. Conversely, when the positional relationship of the pupil's centroid relative to the reference point for the reference eye as viewed in the horizontal direction is opposite to the positional relationship of the redetection target eye relative to the reference eye as viewed in the horizontal direction on the image, the estimating unit 25 may shift the position of the estimated range horizontally toward the side opposite from the reference point.

Further, the estimating unit 25 obtains, based on the shape of the pupil region for the reference eye, the estimated shape of the pupil region to be detected for the redetection target eye.

For example, the estimating unit 25 obtains an elliptical shape having the same horizontal and vertical widths as the horizontal and vertical widths of the pupil region for the reference eye as the estimated shape of the pupil region to be detected for the redetection target eye.

The estimating unit 25 may correct, based on the gaze direction, the estimated shape of the pupil region to be detected.

Generally, the circularity of the pupil region on the image increases as the angle that the gaze direction makes with the line joining the infrared camera 4 to the pupil's centroid decreases, because the pupil is turned closer to the line of sight of the infrared camera 4. Therefore, based on the magnitude relationship between the angles that the gaze directions detected for the left and right eyes, respectively, make with the line joining the infrared camera 4 to the pupil's centroid, the estimating unit 25 can correct the estimated shape of the pupil region to be detected.

In view of the above, the estimating unit 25 calculates a first angle which the gaze direction of the reference eye makes with the line joining the eye to the infrared camera 4 and a second angle which the gaze direction of the redetection target eye makes with the line joining the eye to the infrared camera 4. When the first angle is smaller than the second angle, the estimating unit 25 sets the ratio of the horizontal width to the vertical width of the estimated shape of the pupil region of the redetection target eye smaller than the ratio of the horizontal width to the vertical width of the pupil region of the reference eye. Conversely, when the first angle is larger than the second angle, the estimating unit 25 sets the ratio of the horizontal width to the vertical width of the estimated shape of the pupil region of the redetection target eye larger than the ratio of the horizontal width to the vertical width of the pupil region of reference eye.

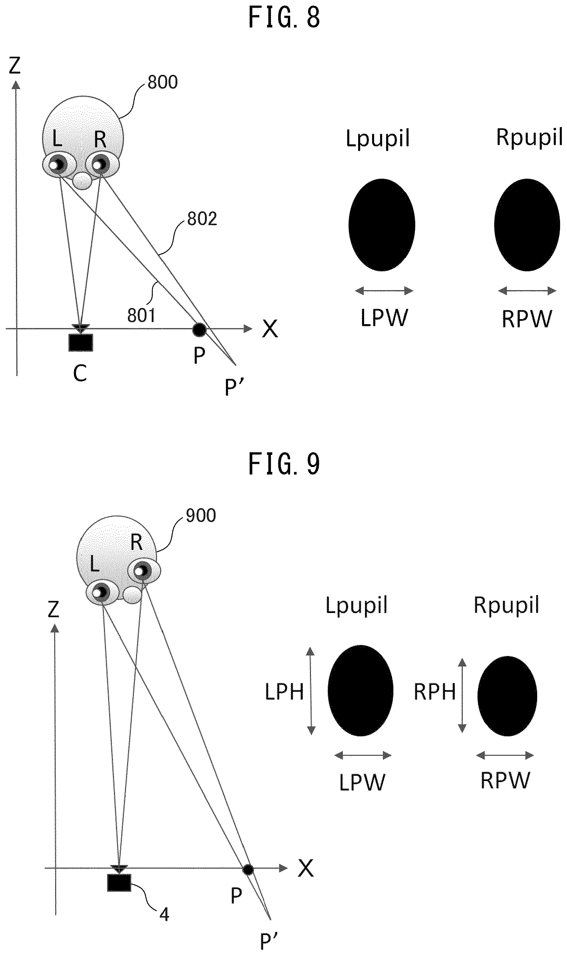

Figure 8 is a diagram illustrating one example of the relationship between the gaze direction and the shape of the pupil region. In the illustrated example, it is assumed that the user 800 is looking at a gaze point P' which is located rightwardly of the infrared camera 4 (indicated at C in Figure 8). In Figure 8, the optical axis direction of the infrared camera 4 is taken to be the Z axis, and the direction perpendicular to the optical axis direction and parallel to the ground surface (i.e., the direction along which the eyes of the user 800 are arranged) to be the X axis. When the position of the gaze point P' is known, the angle ∠CLP' that the gaze direction 801 for the left-side eye L as seen from the infrared camera 4 (i.e., the right eye of the user 800) makes with the line joining the pupil's centroid to the infrared camera 4 is expressed by the following equation.

$$\angle CLP'[\deg] = \tan^{-1}\left(\left|Lx - Cx\right|/\left|Lz - Cz\right|\right) - \tan^{-1}\left(\left|Lx - P'x\right|/\left|Lz - P'z\right|\right) \qquad (1)$$

where CX, LX, and P'X represent the position of the infrared camera 4, the position of the left-side eye, and the position of the gaze point P', respectively, on the X axis. In addition, CZ, LZ, and P'Z represent the position of the infrared camera 4, the position of the left-side eye, and the position of the gaze point P', respectively, on the Z axis.

Likewise, the angle ∠CRP' that the gaze direction 802 for the right-side eye R (i.e., the left eye of the user 800) makes with the line joining the pupil's centroid to the infrared camera 4 is expressed by the following equation.

$$\angle CRP'[\deg] = 180 - \tan^{-1}\left(\left|Rx - Cx\right|/\left|Rz - Cz\right|\right) - \tan^{-1}\left(\left|Lx - P'x\right|/\left|Lz - P'z\right|\right) \qquad (2)$$

where RX represents the position of the right-side eye on the X axis, and RZ represents the position of the right-side eye on the Z axis. Then, the relationship between the horizontal width LPW of the left-side eye's pupil Lpupil and the horizontal width RPW of the right-side eye's pupil Rpupil is expressed by the following equation.

$$\frac{LPW}{RPW} = \frac{\sin(\angle CLP')}{\sin(\angle CRP')} \qquad (3)$$

The estimating unit 25 calculates ∠CLP' and ∠CRP' in accordance with the above equations (1) and (2). LX and RX are calculated from the coordinates of the right and left eyes on the image. P'X is obtained from the positional relationship between the gaze point P' and the infrared camera 4. Then, by substituting the calculated ∠CLP' and ∠CRP' and the horizontal width of the pupil region of the reference eye into the equation (3), the estimating unit 25 can calculate the horizontal width of the estimated shape of the pupil region to be detected. It is considered that the vertical width of the pupil region is substantially the same for both the left-side and right-side eyes. Therefore, the estimating unit 25 need only take the vertical width of the pupil region of the reference eye as the vertical width of the estimated shape of the pupil region to be detected.

The estimating unit 25 may perform processing similar to that performed by the gaze detection unit 27 and may detect the user's gaze direction and gaze position based on the positional relationship between the reference point and the pupil's centroid detected for the reference eye. Then, the estimating unit 25 may calculate the above angle for the redetection target eye by taking the direction pointing from the reference point to the gaze position as the gaze direction. Alternatively, the estimating unit 25 may utilize the immediately previously obtained gaze direction as the gaze direction.

There can also occur a case where the gaze point is located farther from the user than the infrared camera 4. In other words, the distance from the gaze point P' to the user's eye (|P'Z-LZ| or |P'Z-RZ|) is longer than the distance from the infrared camera 4 to the user's eye (|CZ-LZ| or |CZ-RZ|). In this case, the estimating unit 25 may set a tentative gaze point P at the position where the line drawn parallel to the X axis by passing through the position of the infrared camera 4 on the Z axis intersects the gaze direction, and may calculate ∠CLP and ∠CRP instead of ∠CLP' and ∠CRP'. Then, using ∠CLP and ∠CRP, the estimating unit 25 may calculate the horizontal width of the estimated shape of the pupil region to be detected for the redetection target eye in accordance with the equation (3). However, in this case, it is preferable that the estimating unit 25 takes respective values falling within a first offset range centered around the calculated horizontal width to determine the horizontal width of the estimated shape of the pupil region to be detected.

When the distance from the user to the gaze point P' is not known, the estimating unit 25 may take respective values falling within a second offset range centered around the horizontal width of the pupil region of the reference eye to determine the horizontal width of the estimated shape of the pupil region to be detected. In this case, it is preferable to set the second offset range wider than the first offset range.

Further, when the distance from the infrared camera 4 to the user is known in advance, the estimating unit 25 may correct, based on the orientation of the user's face, the vertical width of the estimated shape of the pupil region to be detected. Generally, the size of the pupil region on the image decreases as the distance from the infrared camera 4 to the pupil increases.

Figure 9 is a diagram illustrating one example of the relationship between the gaze direction and the shape of the pupil region when the user's face is oriented at an angle relative to the line of sight of the infrared camera 4. In Figure 9 also, the optical axis direction of the infrared camera 4 is taken to be the Z axis, and the direction perpendicular to the optical axis direction and parallel to the ground surface (i.e., the direction along which the eyes of the user 900 are arranged) to be the X axis. As illustrated in Figure 9, when the user 900 turns his face toward the gaze point P', the eye located on the same side as the gaze point P' is farther from the infrared camera 4 than the eye located on the side opposite from the gaze point P'. In the illustrated example, since the gaze point P' is located rightwardly of the infrared camera 4, the right-side eye is farther from the infrared camera 4 than the left-side eye as seen from the infrared camera 4. As a result, the vertical width RPH and horizontal width RPW of the right-side eye's pupil Rpupil appear smaller than the vertical width LPH and horizontal width LPW of the left-side eye's pupil Lpupil.

Then, the estimating unit 25, for example, detects the orientation of the user's face from the image and, based on the orientation of the face, calculates the ratio between the distance from the infrared camera 4 to the right-side eye and the distance from the infrared camera 4 to the left-side eye. The estimating unit 25 may detect the line joining the midpoint between the inner corners of the two eyes to the midpoint of the lip, for example, as the centerline and may determine the orientation of the face based on the positional relationship of the highest point of the nose relative to the centerline. In this case, in order to detect the lip and the highest point of the nose from the image, the estimating unit 25 may use, for example, a classifier trained to detect a lip and a classifier trained to detect the highest point of a nose. Alternatively, the estimating unit 25 may use other techniques for detecting the lip and the highest point of the nose from the image.

Then, the estimating unit 25 refers, for example, to a mapping table that provides a mapping between the orientation of the face and the ratio, RD {=(|CZ-LZ|)/(|CZ-RZ|)}, of the distance from the infrared camera 4 to the left-side eye (|CZ-LZ|) to the distance from the infrared camera 4 to the right-side eye (|CZ-RZ|). In this way, the estimating unit 25 obtains the ratio RD based on the detected orientation of the face.

Using the ratio RD, the estimating unit 25 corrects the vertical width and horizontal width of the pupil region for the reference eye. For example, when the reference eye is the right-side eye, the estimating unit 25 multiplies the vertical width and horizontal width of the pupil region for the right-side eye by the ratio RD. On the other hand, when the reference eye is the left-side eye, the estimating unit 25 divides the vertical width and horizontal width of the pupil region for the left-side eye by the ratio RD.

Then, the estimating unit 25 takes the corrected vertical width of the pupil region as the vertical width of the estimated shape of the pupil region to be detected. Further, the estimating unit 25 may apply a horizontal width correction such as described above to the horizontal width of the pupil region and may take the corrected horizontal width as the horizontal width of the estimated shape of the pupil region to be detected.

The estimating unit 25 notifies the pupil redetection unit 26 of the estimated range for the redetection target eye and the estimated shape of the pupil region to be detected.

The pupil redetection unit 26, notified of the estimated range for the redetection target eye and the estimated shape of the pupil region to be detected, executes redetection of the pupil region. In this case, the pupil redetection unit 26 detects the pupil region by searching for a region having a shape that matches the estimated shape of the pupil region from within the estimated range set for the redetection target eye.

The pupil redetection unit 26 detects the pupil region by template matching, for example, using a template of the pupil having the same horizontal and vertical widths as those of the estimated shape of the pupil region to be detected. Alternatively, the pupil redetection unit 26 may detect the pupil region by using a double ring filter of an elliptical shape having the same horizontal and vertical widths as those of the estimated shape of the pupil region to be detected.

In this way, by redetecting the pupil region in accordance with the preset estimated range and the estimated shape to be detected, the pupil redetection unit 26 can detect the pupil region with higher accuracy. Furthermore, the pupil redetection unit 26 can detect the pupil region even when the pupil is partially hidden from view by the Purkinje image of the illuminating light source 3 or by an image of the light source reflected from glasses or the like. When the pupil detection unit 23 failed to detect the pupil region for the redetection target eye, the pupil redetection unit 26 may relax the pupil region detection conditions compared with the pupil region detection conditions applied to the pupil detection unit 23. For example, the pupil redetection unit 26 may set the threshold value for the degree of matching obtained by template matching or the threshold value for the output value produced by the double ring filter lower than the threshold value used in the pupil detection unit 23.

The pupil redetection unit 26 calculates the pupil's centroid for the pupil region detected as a result of the redetection. For example, when the pupil region is detected by template matching, the pupil redetection unit 26 takes as the pupil's centroid the centroid of the template that best matches the pupil region. On the other hand, when the pupil region is detected using a double ring filter, the pupil redetection unit 26 takes as the pupil's centroid the centroid of the double ring filter at the position where the double ring filter is located when the pupil region is detected.

There are cases where a portion of the pupil region is hidden from view. For example, when a region growing method is applied starting with the pixel having the minimum luminance in the detected pupil region, the pupil redetection unit 26 obtains a region formed by an aggregate of pixels having luminance values falling within a predetermined range of luminance values starting from the minimum luminance value, and redetects this region as the pupil region. Then, when a portion of the contour of the pupil region is convex toward the center, or when the circularity calculated from the circumferential length and area of the pupil region is less than a predetermined threshold value, then the pupil redetection unit 26 determines that a portion of the pupil region is hidden from view. In such cases, the pupil redetection unit 26 may estimate the position of the pupil's centroid, for example, based on the contour of the unhidden portion of the pupil region.

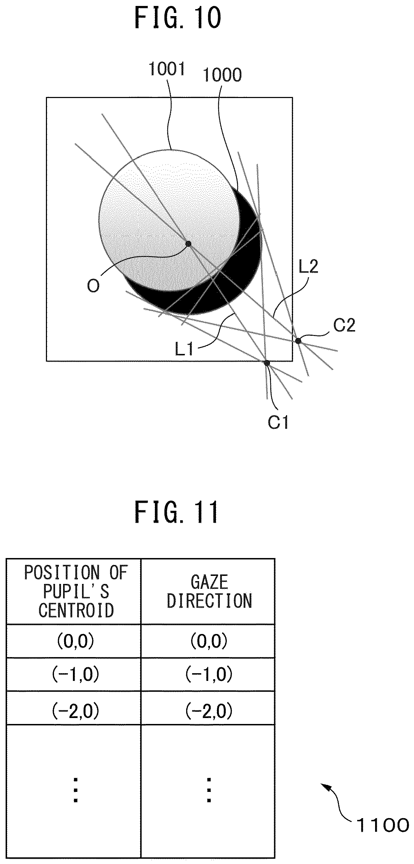

Figure 10 is an explanatory diagram providing an overview of how the position of the pupil's centroid is estimated based on the contour of the unhidden portion of the pupil region. In the example illustrated in Figure 10, a portion of the pupil region 1000 is hidden from view by a blocking object (for example, an image of the light source reflected from glasses) 1001. Then, the pupil redetection unit 26 sets a plurality of pairs of points on the contour of the outwardly convex portion of the pupil region 1000. Then, for each pair, the pupil redetection unit 26 obtains a point of intersection C1 or C2 at which the lines tangent to the contour at the two points intersect, and calculates a bisector L1 or L2 of the angle that the tangent lines make with each other at the point of intersection. The pupil redetection unit 26 estimates the pupil's centroid, O, by calculating the point of intersection of the bisectors L1 and L2 calculated for the respective pairs.

The pupil redetection unit 26 notifies the gaze detection unit 27 of the position of the pupil's centroid detected as a result of the redetection.

The gaze detection unit 27 detects the user's gaze direction and gaze position based on the position of the pupil's centroid detected for each of the user's left and right eyes.

Since the surface of the cornea is substantially spherical in shape, the position of the Purkinje image of the light source remains essentially unchanged even when the gaze direction changes. On the other hand, the pupil's centroid changes as the gaze direction changes. Therefore, the gaze detection unit 27 can detect the gaze direction by obtaining the position of the pupil's centroid relative to the centroid of the Purkinje image of the illuminating light source 3.

In the present embodiment, the gaze detection unit 27 obtains the position of the pupil's centroid relative to the centroid of the Purkinje image of the illuminating light source 3 for each of the left and right eyes. More specifically, the gaze detection unit 27 obtains the relative position of the pupil's centroid, for example, by subtracting the horizontal and vertical coordinates of the centroid of the Purkinje image from the horizontal and vertical coordinates of pupil's centroid. Then, the gaze detection unit 27 determines the gaze direction for each of the left and right eyes by referring to a mapping table that provides a mapping between the relative position of the pupil's centroid and the gaze direction.

Figure 11 is a diagram illustrating one example of the mapping table. Each entry in the left-hand column of the mapping table 1100 carries the coordinates of the position of the pupil's centroid relative to the centroid of the Purkinje image of the illuminating light source 3. Each entry in the right-hand column of the mapping table 1100 carries the gaze direction corresponding to the coordinates of the relative position of the pupil's centroid carried in the left-hand entry. In the illustrated example, the gaze direction is expressed in terms of the horizontal and vertical angular differences relative to the reference gaze direction which is, in this case, the gaze direction when the user is gazing at a designated reference point (for example, the center of the display screen of the display unit 2 or the mounting position of the infrared camera 4). The coordinates of the relative position of the pupil's centroid are expressed in units of pixels on the image.

Alternatively, the gaze detection unit 27 may detect the gaze direction for each of the left and right eyes, based on the positional relationship between a reference point other than the Purkinje image of the illuminating light source 3 and the pupil's centroid. For example, when the reference point is a feature point of the eye, such as the inner corner or the outer corner of the eye or the midpoint between the inner and outer corners of the eye, the gaze detection unit 27 can detect the gaze direction based on the position of the pupil's centroid relative to such a reference point. However, in this case, since the relationship between the eye's feature point and the pupil's centroid changes with the orientation of the user's face, it is preferable that the gaze detection unit 27 also detects the orientation of the user's face as earlier described and corrects the gaze direction by considering the orientation of the user's face.

Further, the gaze detection unit 27 detects the user's gaze position based on the detected gaze direction. In the present embodiment, the gaze detection unit 27 determines the gaze position by referring to a gaze position table that provides a mapping between the gaze direction and the gaze position. The relationship between the gaze direction and the gaze position changes according to the distance between the gaze position (for example, the display screen of the display unit 2) and the user. The horizontal width of the face on the image also changes according to the distance between the infrared camera 4 and the user. Since the infrared camera 4 is disposed in the vicinity of the display unit 2, it can be assumed that the distance between the infrared camera 4 and the user and the distance between the user's gaze position and the user are approximately equal.

Therefore, a plurality of gaze position tables are constructed in advance for various distances (for example, 1 m, 2 m, etc.) expected between the infrared camera 4 and the user of the digital signage system 1, and stored in the storage unit 7. Each gaze position table is associated with a range within which the horizontal width of the face region on the image is expected to be contained according to the expected distance between the infrared camera 4 and the user. The gaze detection unit 27 selects the gaze position table associated with the range within which the horizontal width of the face region most recently received from the eye detection unit 21 is contained. Then, the gaze detection unit 27 obtains the gaze position corresponding to the gaze direction by referring to the selected gaze position table.

When the digital signage system 1 is equipped with a range sensor such as a depth camera, the gaze detection unit 27 need only select the gaze position table according to the distance to the user detected by the range sensor.

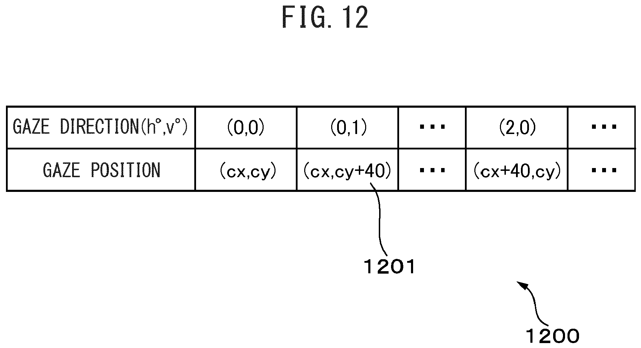

Figure 12 is a diagram illustrating one example of the gaze position table. The upper row in the gaze position table 1200 carries the gaze direction. Each entry in the gaze position table 1200 carries the coordinates of the gaze position corresponding to the gaze direction carried in the upper row; the coordinates are expressed in predetermined units (for example, in units of pixels on the display screen of the display unit 2 or in millimeters). For example, entry 1201 in the gaze position table 1200 indicates that the gaze position is (cx, cy+40) when the gaze direction is 0° in the horizontal direction and 1° in the vertical direction. In the illustrated example, cx and cy are the coordinates of the gaze position when the gaze direction is (0, 0), i.e., the coordinates of the reference gaze position, for example, the horizontal and vertical coordinates in a vertical plane at the mounting position of the infrared camera 4.

The gaze detection unit 27 determines the user's gaze position by taking the average value of the gaze positions obtained for the left and right eyes. Then, the gaze detection unit 27 may correct the gaze direction for each of the left and right eyes by referring to the gaze position table based on the average value of the gaze positions obtained for the left and right eyes. Then, the control unit 8 stores the user's gaze position and the gaze direction of each of the left and right eyes in the storage unit 7.

Alternatively, the gaze detection unit 27 may store the gaze position and the gaze direction for each of the left and right eyes in the storage unit 7.

Then, by tracking the user's gaze position obtained as the average value of the gaze positions of the two eyes, or the gaze position calculated for one of the eyes, the control unit 8 identifies the position or range where the gaze position has continued to stay for more than a predefined period of time. The control unit 8 then performs processing appropriate to that position or range. For example, the control unit 8 may cause the display unit 2 to display information concerning the icon or content displayed in that position or range on the display unit 2.

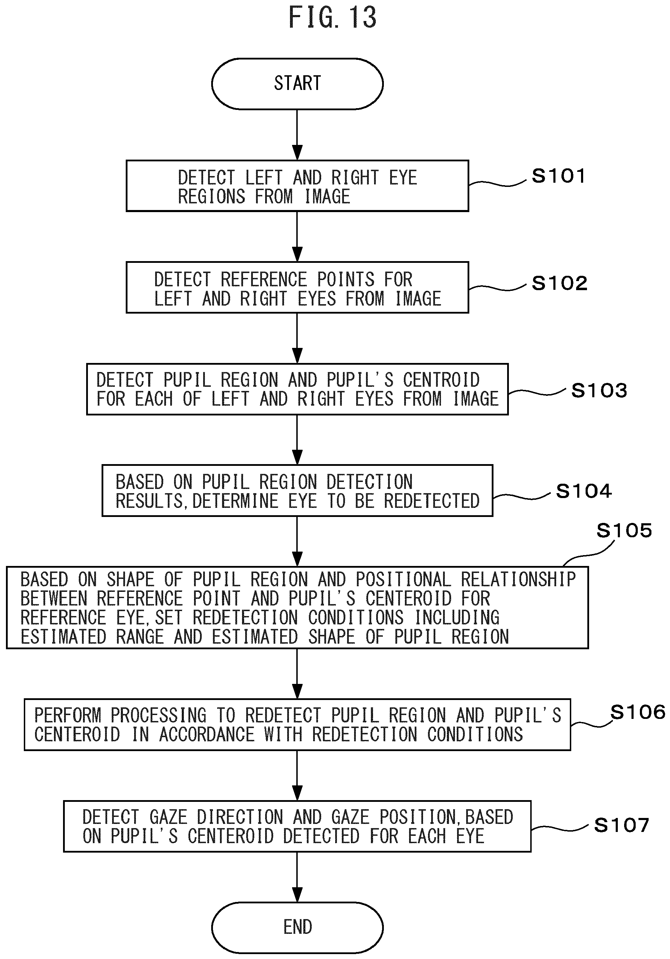

Figure 13 illustrates an operation flowchart of the gaze detection process executed by the control unit 8. The control unit 8 executes the gaze detection process in accordance with the following operation flowchart each time an image is acquired.

The eye detection unit 21 detects left and right eye regions on the image acquired from the infrared camera 4 (step 5101). The reference point detection unit 22 detects the reference point in each of the left and right eye regions (step S102). Then, the pupil detection unit 23 detects the pupil region and the pupil's centroid in each of the left and right eye regions (step 5103).

The redetection determining unit 24 determines the redetection target eye, based on the pupil region detection results for the left and right eyes (step S104). Then, based on the shape of the pupil region and the positional relationship between the reference point and the pupil's centroid for the reference eye, the estimating unit 25 sets redetection conditions including the estimated range for the redetection target eye and the estimated shape of the pupil region to be detected (step S105).

The pupil redetection unit 26 performs processing to redetect the pupil region and the pupil's centroid in accordance with the redetection conditions (S106). The gaze detection unit 27 obtains the user's gaze direction and gaze position, based on the position of the pupil's centroid detected for each of the left and right eyes (step 5107). Then, the control unit 8 terminates the gaze detection process.

As has been described above, based on the shape of the pupil region and the positional relationship between the reference point and the pupil's centroid detected for one of the left and right eyes contained in the image, the gaze detection apparatus sets the redetection conditions for redetecting the pupil region for the other eye. Accordingly, the gaze detection apparatus can estimate the position of the pupil's centroid for the other eye, even when a portion of the pupil region is hidden from view. When the pupil regions have been detected successfully for both of the eyes, the gaze detection apparatus redetects the pupil region for the eye considered lower in detection accuracy, by using the redetection conditions set based on the pupil region detected for the eye considered higher in detection accuracy. In this way, the gaze detection apparatus can enhance the detection accuracy of the pupil region for the eye considered lower in detection accuracy.

According to a modified example, the estimating unit 25 may obtain only one of the conditions, the estimated range or the estimated shape of the pupil region, as the redetection condition. Then, the pupil redetection unit 26 may redetect the pupil region by using only one of them as the redetection condition. For example, when only the estimated shape of the pupil region is used as the redetection condition, the pupil redetection unit 26 may search the entire eye region and detect a region having a shape that matches the estimated shape as the pupil region. For example, due to the presence of eyelashes, glasses, or a shadow, a false dark region similar in shape to the pupil may appear in the image containing the eye. According to the gaze detection apparatus of this example, since the pupil region is redetected based on the estimated shape of the pupil region, the chance of erroneously detecting the false dark region as the pupil can be reduced. On the other hand, when only the estimated range of the pupil region is used as the redetection condition, the pupil redetection unit 26 need only perform processing to redetect the pupil region within the estimated range, without specifically limiting the shape of the pupil region to be detected.

The gaze detection apparatus according to the above embodiment or its modified example is not limited in its application to the digital signage system described above, but may be used in a vehicle driving assisting system, a computer input assisting system, etc. Further, depending on the application, the gaze detection apparatus need only detect the user's gaze direction and need not detect the gaze position.

A computer program for implementing the various functions of the control unit in the gaze detection apparatus according to the above embodiment or its modified example may be provided in the form recorded on a computer readable recording medium such as a magnetic recording medium or an optical recording medium. The recording medium here does not include a carrier wave.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of superiority and inferiority of the invention. Although the embodiments of the present invention have been described in detail, it should be understood that the various changes, substitutions, and alterations could be made hereto without departing from the spirit and scope of the invention.

**Claims**

1. A gaze detection apparatus comprising:

an eye detection unit which, for each of the eyes, detects an eye region containing the eye from an image representing both eyes of a user;
a pupil detection unit which, for one of the eyes, detects a pupil region containing a pupil from the eye region;
an estimating unit which, based on the pupil region detected for the one eye, obtains an estimated shape of the pupil region on the image for the other one of the eyes;
a pupil redetection unit which detects the pupil region by searching for a region having a shape that matches the estimated shape from within the eye region detected for the other eye; and
a gaze detection unit which detects the user's gaze direction based on a position of the pupil region detected for each of the eyes.

2. The gaze detection apparatus according to claim 1, wherein the estimating unit sets, when a first angle which the gaze direction detected for the one eye makes with a line joining the one eye to the image capturing unit is smaller than a second angle which the gaze direction estimated for the other eye makes with a line joining the other eye to the image capturing unit, the ratio of a width of the estimated shape of the pupil region of the other eye, measured in a first direction along which the eyes are arranged, to the width thereof measured in a second direction perpendicular to the first direction, smaller than the ratio of the width of the pupil region of the one eye, measured in the first direction, to the width thereof measured in the second direction, while on the other hand, the estimating unit sets, when the first angle is larger than the second angle, the ratio of the width of the estimated shape of the pupil region of the other eye, measured in the first direction, to the width thereof measured in the second direction, larger than the ratio of the width of the pupil region of the one eye, measured in the first direction, to the width thereof measured in the second direction.

3. The gaze detection apparatus according to claim 1, further comprising a reference point detection unit which detects, for each of the eyes, a reference point whose position does not change with the user's gaze direction, and wherein the estimating unit obtains, based on a positional relationship between the pupil region and the reference point detected for the one eye and on the reference point detected for the other eye, an estimated range within which the pupil region for the other eye is estimated to be located, and the pupil redetection unit detects the pupil region by searching for a region having a shape that matches the estimated shape from within the estimated range.

4. The gaze detection apparatus according to claim 3, further comprising an illuminating light source which illuminates both of the eyes, and wherein the reference point detection unit detects a position of a corneal reflection image of the illuminating light source as the reference point for each of the eyes, and the estimating unit estimates, based on the positional relationship between the pupil region and the reference point detected for the one eye, a positional relationship between the gaze direction and the illuminating light source, as viewed in a first direction along which the eyes are arranged, and obtains the estimated range based on the estimated positional relationship.

5. The gaze detection apparatus according to claim 4, wherein the estimating unit sets, when the positional relationship of the gaze direction relative to the illuminating light source as viewed in the first direction is identical to the positional relationship of the other eye relative to the one eye as viewed in the first direction, the estimated range so that a distance from the reference point for the other eye to a center of the estimated range is longer than a distance from the reference point for the one eye to a centroid of the pupil region along a direction directed away from the reference point for the one eye and toward the pupil region.

6. The gaze detection apparatus according to claim 3, wherein the reference point detection unit detects, for each of the eyes, a position of the eye's inner corner as the reference point, and the estimating unit sets the estimated range so that a distance from the reference point for the other eye to a center of the estimated range is shorter as a distance from the reference point for the one eye to a centroid of the pupil region is longer.

7. The gaze detection apparatus according to claim 3, wherein the reference point detection unit detects, for each of the eyes, a midpoint between the eye's inner and outer corners as the reference point, and

the estimating unit sets, when the positional relationship of the pupil region relative to the reference point for the one eye, as viewed in a first direction along which the eyes are arranged, is identical to the positional relationship of the other eye relative to the one eye, the estimated range so that a distance from the reference point for the other eye to a center of the estimated range is shorter than a distance from the reference point for the one eye to a centroid of the pupil region.

8. The gaze detection apparatus according to any one of claims 1 to 7, wherein the pupil detection unit detects the pupil region from the eye region for each of the eyes, and calculates an evaluation score indicating degree of certainty of the detected pupil region, and wherein the gaze detection apparatus further comprises:

   a redetection determining unit which determines that, of the two eyes, the eye for which the evaluation score is lower corresponds to the other eye.

9. A gaze detection method comprising:

   generating an image representing both eyes of a user;
   detecting, for each of the eyes, an eye region containing the eye from the image;
   detecting, for one of the eyes, a pupil region containing a pupil from the eye region;
   obtaining, based on the pupil region detected for the one eye, an estimated shape of the pupil region on the image for the other one of the eyes;
   detecting the pupil region by searching for a region having a shape that matches the estimated shape from within the eye region detected for the other eye; and
   detecting the user's gaze direction based on a position of the pupil region detected for each of the eyes.

10. A gaze detection computer program for causing a computer to execute a process comprising:

   detecting, from an image representing both eyes of a user, for each of the eyes, an eye region containing the eye;
   detecting, for one of the eyes, a pupil region containing a pupil from the eye region;
   obtaining, based on the pupil region detected for the one eye, an estimated shape of the pupil region on the image for the other one of the eyes;
   detecting the pupil region by searching for a region having a shape that matches the estimated shape from within the eye region detected for the other eye; and
   detecting the user's gaze direction based on a position of the pupil region detected for each of the eyes.

# FIG. 1

# FIG. 2

# FIG. 3

CONTROL UNIT

| EYE DETECTION UNIT | 21 |

| REFERENCE POINT DETECTION UNIT | 22 |

| PUPIL DETECTION UNIT | 23 |

| REDETECTION DETERMINING UNIT | 24 |

| ESTIMATING UNIT | 25 |

| PUPIL REDETECTION UNIT | 26 |

| GAZE DETECTION UNIT | 27 |

8

# FIG. 4

# FIG. 5

# FIG. 6

602　601　611　612

# FIG. 7

701　702　700

701　702　710

721　723　724

721　723　710

722

# FIG. 8

# FIG. 9

# FIG. 10

# FIG. 11

| POSITION OF PUPIL'S CENTROID | GAZE DIRECTION |
|---|---|
| (0,0) | (0,0) |
| (-1,0) | (-1,0) |
| (-2,0) | (-2,0) |
| ⋮ | ⋮ |

1100

# FIG. 12

| GAZE DIRECTION(h°,v°) | (0,0) | (0,1) | ・・・ | (2,0) | ・・・ |
|---|---|---|---|---|---|
| GAZE POSITION | (cx,cy) | (cx,cy+40) | ・・・ | (cx+40,cy) | ・・・ |

1201

1200

# FIG. 13

START

DETECT LEFT AND RIGHT EYE
REGIONS FROM IMAGE — S101

DETECT REFERENCE POINTS FOR
LEFT AND RIGHT EYES FROM IMAGE — S102

DETECT PUPIL REGION AND PUPIL'S CENTROID
FOR EACH OF LEFT AND RIGHT EYES FROM IMAGE — S103

BASED ON PUPIL REGION DETECTION
RESULTS, DETERMINE EYE TO BE REDETECTED — S104

BASED ON SHAPE OF PUPIL REGION AND POSITIONAL RELATIONSHIP
BETWEEN REFERENCE POINT AND PUPIL'S CENTROID FOR
REFERENCE EYE, SET REDETECTION CONDITIONS INCLUDING
ESTIMATED RANGE AND ESTIMATED SHAPE OF PUPIL REGION — S105

PERFORM PROCESSING TO REDETECT PUPIL REGION AND PUPIL'S
CENTEROID IN ACCORDANCE WITH REDETECTION CONDITIONS — S106

DETECT GAZE DIRECTION AND GAZE POSITION, BASED
ON PUPIL'S CENTEROID DETECTED FOR EACH EYE — S107

END

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012024154 A

**Non-patent literature cited in the description**

- **P. FELZENSZWALB et al.** A Discriminatively Trained, Multiscale, Deformable Part Model. *Computer Vision and Pattern Recognition 2008, IEEE Conference on Year,* 2008, 1-8